# FASCICULE DE BREVET EUROPEEN

(11) **EP 2 512 432 B1**
(45) Date de publication et mention de la délivrance du brevet: **18.07.2018**
(21) Numéro de dépôt: 10792983.8
(22) Date de dépôt: 08.11.2010
(51) Int. Cl.: A61K 31/715, A61P 19/02, C12N 5/077, A61K 9/00

(54) **COMPOSITIONS INJECTABLES A USAGE INTRA-ARTICULAIRE ASSOCIANT UN AGENT DE VISCOSUPPLEMENTATION ET UN MILIEU DE CROISSANCE DES FIBROBLASTES**
INJIZIERBARE ZUSAMMENSETZUNGEN ZUR INTRAARTIKULÄREN VERWENDUNG MIT VISKOSUPPLEMENTATION UND EINEM FIBROBLASTENWACHSTUMSMEDIUM
INJECTABLE COMPOSITIONS FOR INTRA-ARTICULAR USE COMBINING A VISCOSUPPLEMENTATION AGENT AND A FIBROBLAST GROWTH MEDIUM

(30) Priorité: 18.12.2009 FR 0959205
(43) Date de publication de la demande: 24.10.2012
(73) Titulaire: Thorel, Jean-Noël, 75014 Paris (FR)
(72) Inventeur: GATTO, Hugues, 06570 Saint Paul De Vence (FR)
(74) Mandataire: Cabinet Laurent & Charras
(86) Numéro de dépôt international: PCT/FR2010/052397
(87) Numéro de publication internationale: WO 2011/073546

(56) Documents cités:
- WO-A1-98/08934
- WO-A2-02/39948
- WO-A2-03/072128
- WO-A2-2007/119213

## Description

La présente invention s'inscrit dans le développement de solutions injectables au niveau intra-articulaire pour le traitement des dégénérescences articulaires, en particulier de l'arthrose.

Elle propose d'associer un agent de viscosupplémentation, tel que l'acide hyaluronique ou l'un de ses sels, avec un milieu de croissance des fibroblastes de composition bien déterminée, et éventuellement un autre polysaccharide, avantageusement d'origine naturelle.

### Etat antérieur de la technique

Au niveau de certaines articulations des membres, les extrémités osseuses en regard, protégées par les cartilages articulaires, sont enchassées dans une capsule tapissée par un tissu conjonctif, appelé membrane synoviale.

Le liquide synovial est le fluide visqueux qui remplit la cavité articulaire, et qui est composé d'acide hyaluronique (AH), sécrété par les cellules de type fibroblaste de la membrane synoviale (synoviocytes), et de liquide interstitiel filtré du plasma sanguin. Le liquide synovial a pour fonction de réduire la friction en lubrifiant l'articulation, d'absorber les chocs, de fournir de l'oxygène et des nutriments aux chondrocytes du cartilage articulaire et d'éliminer, de ces derniers, le dioxyde de carbone et les déchets métaboliques, dans la mesure où le cartilage n'est pas vascularisé.

L'arthrose est une affection articulaire dégénérative fréquente, d'étiologie plurifactorielle, associée à une perte de matière au niveau des cartilages articulaires. Au cours du développement de l'affection, une réduction de la concentration et du poids moléculaire de l'AH présent dans le liquide synovial est observée. Ce phénomène s'explique d'une part par une baisse de la synthèse endogène d'acide hyaluronique et d'autre part par l'inflammation qui génère des radicaux libres, responsables d'une dégradation radicalaire.

Ces altérations entraînent une réduction des propriétés viscoélastiques de l'AH et conduisent progressivement à la perte de son rôle essentiel de protection de l'articulation. Erosion du cartilage, présence de fragments de cartilage ou d'os dans la cavité articulaire, douleur, raideur peuvent résulter de ces altérations.

La viscosupplémentation est une option thérapeutique bien établie qui consiste à injecter de l'AH dans l'articulation concernée pour aider à mieux lubrifier l'articulation, augmenter la mobilité et réduire la douleur. Selon le degré de sévérité de l'arthrose, des séries de 3 à 5 injections hebdomadaires, au niveau du genou par exemple, procurent une efficacité de 6 mois à 1 an chez une majorité de patients. Cette option thérapeutique est extrêmement utile, notamment pour les patients qui ne tolèrent pas, ou ne répondent plus aux traitements conventionnels comme les anti-inflammatoires et antalgiques oraux, mais qui ne justifient pas encore d'un traitement prothétique.

L'amélioration des solutions de viscosupplémentation a concerné, jusqu'à présent, l'augmentation du temps de résidence de l'AH dans l'articulation, dans le but d'augmenter son efficacité.

Ainsi, des procédés de modification chimique de l'AH, de réticulation de l'AH (WO 2007/070547), ou la combinaison de l'AH avec un polyol (WO 2009/024670), ont été décrits pour ralentir la dégradation radicalaire, thermique ou mécanique du gel d'AH *in vivo.*

Toutefois, la rémanence intra-articulaire des produits de viscosupplémentation à base d'AH, modifié ou non, dont la demi-vie est de quelques jours, reste très inférieure à la durée de leur efficacité thérapeutique. En effet, celle-ci repose sur plusieurs rôles cumulés :
- rôle d'amortisseur direct des chocs sur le cartilage ;
- propriétés de captation des débris intra-articulaires diminuant ainsi leur effet abrasif ;
- protection contre les cellules de l'inflammation et les enzymes qu'elles sécrètent ; et
- possible action directe sur les récepteurs de la douleur.

Il existe donc un besoin évident de développer de nouvelles solutions thérapeutiques pour le traitement des dégénérescences articulaires, notamment de l'arthrose.

### Description de l'invention

Dans ce cadre, le Demandeur s'est inscrit dans une démarche totalement nouvelle. En effet, la présente invention propose d'agir à deux niveaux distincts pour rétablir un bon fonctionnement des articulations, notamment pour le traitement des dégénérescences articulaires, en particulier de l'arthrose.

Ainsi, la présente invention concerne une composition injectable, associant à la fois un agent de viscosupplémentation et d'autre part, un milieu de croissance des fibroblastes tel que défini dans les revendications, destiné à revitaliser les composants cellulaires des tissus conjonctifs de l'articulation, notamment synoviocytes et chondrocytes, et par conséquent à assurer leur régénération cellulaire ainsi que la stimulation de leurs synthèses endogènes (en acide hyaluronique et GAGs, éléments constitutifs et fonctionnels fondamentaux des articulations).

Plus précisément, les composants susceptibles de jouer le rôle d'agent de viscosupplémentation dans une composition d'injection intra-articulaire selon l'invention sont choisis dans la liste suivante : l'acide hyaluronique, la chondroïtine sulfate, le kératane, le kératane sulfate, l'héparine, la cellulose et ses dérivés, le chitosane, les xanthanes, le galactomannane, les alginates et leurs sels respectifs.

En pratique, l'agent de viscosupplémentation couramment mis en oeuvre dans cette application intra-articulaire est l'acide hyaluronique ou l'un de ses sels. C'est pourquoi dans un mode de réalisation particulier, la composition visée ne contient que l'acide hyaluronique ou l'un de ses sels en tant qu'agent de viscosupplémentation en association avec le milieu de croissance des fibroblastes.

Dans un mode de réalisation alternatif, l'acide hyaluronique ou l'un de ses sels est prévu comme agent de viscosupplémentation principal et combiné, dans la composition visée, à au moins un autre polysaccharide, avantageusement d'origine naturelle pour assurer son caractère biocompatible et non immunogène. Cet autre polysaccharide est avantageusement un glycosaminoglycane polysoufré - notamment la chondroïtine sulfate, le kératane, le kératane sulfate -, ou encore l'héparine, la cellulose et ses dérivés, le chitosane, les xanthanes, le galactomannane, les alginates et leurs sels respectifs. La composition contient en outre le milieu de croissance des fibroblastes, et éventuellement d'autres constituants.

En pratique, il s'agit donc de combiner une action mécanique de lubrification et de protection de l'articulation à une action trophique de relance fibroblastique soutenant les synthèses cellulaires au niveau de la membrane synoviale et du cartilage articulaire. La première action est assurée par l'agent de viscosupplémentation, avantageusement de l'acide hyaluronique - réticulé ou non, ou un des ses sels-, éventuellement en combinaison avec un ou plusieurs autres polysaccharides d'origine naturelle. La seconde action est assurée par le milieu de croissance des fibroblastes tel qu'il va être défini.

De manière connue, l'acide hyaluronique mis en oeuvre dans le cadre de la présente invention peut se présenter sous différentes formes : des sels, des dérivés tels que des esters ou des amides, sous forme linéaire ou bien réticulés chimiquement (« cross-linked »). Toutes ces formes sont envisageables dans le cadre de la présente invention. Si la réticulation permet d'augmenter la durée de vie des molécules d'acide hyaluronique dans l'organisme, ces modifications affectent cependant ses caractéristiques physico-chimiques, ses propriétés biologiques et son immunogénicité potentielle.

Tout comme mentionné pour l'acide hyaluronique, le ou les polysaccharides avantageusement d'origine naturelle, peuvent être réticulés ou non réticulés, greffés ou non greffés, selon les techniques de réticulation et greffage décrites dans l'art antérieur.

Dans la recherche d'une solution technique aussi neutre que possible pour les structures articulaires, dite « biomimétique », l'acide hyaluronique non réticulé, ainsi que ses sels physiologiquement acceptables, est privilégié pour le premier composant dans la mesure où cette molécule est un constituant naturel du liquide synovial. On désigne par sels d'acide hyaluronique physiologiquement acceptables, notamment les sels de sodium et de potassium, ainsi que leur mélange.

De manière avantageuse, l'agent de viscosupplémentation, avantageusement l'acide hyaluronique, est présent dans la composition à une concentration comprise entre 1 et 100 mg/ml, avantageusement comprise entre 10 et 25 mg/ml.

Le deuxième constituant essentiel de la composition selon l'invention est donc un milieu de croissance des fibroblastes tel que défini dans les revendications. Dans le cadre de la présente invention, un milieu de croissance des fibroblastes est défini comme un milieu complet permettant non seulement le maintien des fibroblastes en vie, mais stimulant également leur multiplication et leurs synthèses cellulaires (composants de la matrice extracellulaire et du liquide synovial).

La mise en oeuvre d'un test fonctionnel de croissance permet de déterminer si un milieu donné correspond à un milieu de croissance des fibroblastes selon l'invention. En particulier, un test fonctionnel adapté bien connu de l'homme du métier est le test d'observation de la densité des cellules vivantes par méthode colorimétrique, mettant en oeuvre le réactif WST-1 et une lecture à 450 nm (Berridge, M. V. et al. (1996): The Biochemical and Cellular Basis of Cell Proliferation Assays That Use Tetrazolium Salts. Biochemica 4, 15-19.)

Un milieu de croissance des fibroblastes est par exemple disponible commercialement : il s'agit du milieu de culture standard DMEM (Sigma) supplémenté en facteur de croissance cellulaire SVF (sérum de veau foetal), à hauteur de 10% en masse.

De manière générale, de tels milieux contiennent des extraits d'origine animale ou cellulaire qui ont effectivement un effet stimulateur sur la croissance des fibroblastes, mais qui présentent l'inconvénient de ne pas avoir une composition déterminée ou de contenir des éléments exogènes non traçables tels que le SVF, les extraits de tige pituitaire de boeuf, les facteurs de croissance cellulaires EGF (« epidermal growth factor »), FGF (« fibroblast growth factors »), l'insuline ou la choléra toxine, l'hydrocortisone, la pipérazine, ... Le milieu de croissance des fibroblastes mis en oeuvre dans le cadre de la présente invention ne contient pas de facteur de croissance cellulaire ou d'extrait biologique d'origine animale ou cellulaire, en particulier si ceux-ci ne sont pas tracés ou traçables et/ou ne sont pas de composition déterminée.

L'expression «non tracé » ou « non traçable » signifie que la source de la matière biologique en question et/ou le traitement subi par celle-ci ne peuvent pas être établis ou contrôlés.

En pratique, ledit milieu est avantageusement dépourvu (exempt) d'extrait biologique d'origine animal ou cellulaire, de composé ou facteur de croissance cellulaire, ou d'une hormone.

Dans un mode de réalisation privilégié, il s'agit donc d'introduire par injection intra-articulaire un milieu de croissance de fibroblastes aussi compatible que possible avec l'environnement naturel des articulations, à savoir un milieu comprenant des constituants biomimétiques et/ou biocompatibles (éléments biologiques présents naturellement dans l'organisme ou neutres vis-à-vis de celui-ci, n'induisant pas de réactions allergiques ou inflammatoires). De fait, ce milieu comprend avantageusement des composants de la substance fondamentale des tissus conjonctifs.

Un tel milieu permet de fournir spécifiquement aux fibroblastes un apport nutritionnel optimisé sous la forme de vitamines, d'oligoéléments, d'acides aminés, de sels minéraux, de sucres simples (tels que glucose, ribose, désoxyribose) et/ou complexes (tels que HA), et des facteurs de croissance naturels sous la forme de constituants d'acides nucléiques (bases azotées et pentoses nécessaires à la formation des nucléotides, ainsi que nucléosides). Il présente en outre avantageusement des caractéristiques de pH (entre 6,5 et 7,9, avantageusement entre 7,4 et 7,6 et d'osmolarité (entre 280 et 450 mOsm, avantageusement entre 300 et 350 mOsm) physiologiques.

A noter que l'acide hyaluronique peut être à la fois un ingrédient du milieu de croissance et l'agent de viscosupplémentation. La différence réside à la fois dans la forme de l'AH (nécessairement un sel physiologique de hyaluronate dans le milieu) et par sa quantité (quantités plus faibles dans le milieu).

Pour stimuler la croissance des fibroblastes, un tel milieu peut être enrichi à l'aide d'une substance exogène à l'organisme, mais d'origine naturelle, traçable et de composition bien déterminée. Une substance répondant à cette définition est par exemple un mélange de peptides extraits du lait, ou complexe MPC (« Milk Peptide Complex »), obtenu par précipitations successives du lait puis séparation de certaines protéines soumises à hydrolyse enzymatique.

Cette substance se présentant sous forme d'une poudre déshydratée est avantageusement ajoutée au milieu à hauteur de 0,5 à 5 mg/ml, encore plus avantageusement de 4 à 5 mg/ml. Le milieu complexe répondant à une telle définition a été développé par le Demandeur et correspond à l'association d'une soixantaine de composants en quantités précisément définies comme suit :

| DENOMINATION INTERNATIONALE NOMENCLATURE COSMETIQUE INGREDIENT (INCI) | *CONCENTRATION FINALE* Solution 1 X (en mg/l) |
|---|---|
| EAU | q.s.p. 1 litre |
| CHLORURE DE SODIUM | 5000 à 8000 |
| L-GLUTAMINE ou L-ALANYL-GLUTAMINE | 100 à 3000 |
| SODIUM BICARBONATE | 0 à 2000 |
| D-GLUCOSE | 2000 à 5000 |
| L-ARGININE HCL | 300 à 500 |
| SODIUM ACETATE | 200 à 450 |
| DISODIUM PHOSPHATE Na₂HPO4 | 100 à 1500 |
| L-LEUCINE | 50 à 200 |
| L-SERINE | 50 à 200 |
| CHLORURE DE MAGNESIUM MgCl₂, 6H₂O | 50 à 200 |
| CHLORURE DE POTASSIUM | 50 à 200 |
| L-VALINE | 20 à 150 |
| SODIUM PYRUVATE | 10 à 75 |
| L-LYSINE HCL | 10 à 75 |
| L-HISTIDINE HCL,H₂O | 10 à 75 |
| L-CYSTEINE HCL H₂O | 10 à 75 |
| ADENINE (HCl) | 5 à 50 |
| L-THREONINE | 5 à 50 |
| CHLORURE DE CALCIUM CaCl₂, 2H₂O | 0 à 22,5 |
| MYO-INOSITOL | 5 à 50 |
| ACIDE L-GLUTAMIQUE | 15 à 75 |
| L-ASPARAGINE H₂O | 15 à 75 |
| L-METHIONINE | 10 à 50 |
| L-TYROSINE 2Na₂ 2H₂O | 10 à 50 |
| L-PHENYLALANINE | 2 à 20 |
| L-TRYPTOPHANE | 2 à 20 |
| L-ALANINE | 5 à 30 |
| GLYCINE | 5 à 30 |
| L-ISOLEUCINE | 5 à 30 |
| ACIDE L-ASPARTIQUE | 10 à 50 |
| SODIUM SULFATE | 1 à 10 |
| SULFATE FERREUX FeSO₄, 7H₂O | 1 à 10 |
| ACIDE FOLIQUE | 1 à 5 |
| THYMIDINE | 0,1 à 3 |
| CYANOCOBALAMINE | 0,1 à 3 |
| D-CALCIUM PANTOTHENATE | 1 à 5 |
| THIAMINE HCL | 1 à 5 |
| ACIDE THIOCTIQUE | 0,1 à 1 |
| ZINC SULFATE ZnSO₄,7H₂O | 0,05 à 0,5 |
| SODIUM SILICATE NA₂SIO₃,4H₂O | 0,05 à 0,5 |
| PYRIDOXINE HCL | 0,5 à 3 |
| NIACINAMIDE (NICOTINAMIDE) | 0,5 à 3 |
| RIBOFLAVINE | 0,05 à 0,5 |
| d-BIOTIN | 0,01 à 0,05 |
| SULFATE DE CUIVRE CuSO₄,5H₂O | 0 à 0,005 |
| AMMONIUM MOLYBDATE (NH4)₆MO7O₂₄,4H₂O | 0 à 0,005 |
| AMMONIUM VANADATE NH₄VO₃ | 0 à 0,001 |
| CHLORURE DE MANGANESE MnCl₂, 4H₂O | 0 à 0,0001 |
| HYALURONATE DE SODIUM | 100 à 1000 |
| L-PROLINE | 10 à 100 |
| HYDROXYPROLINE | 10 à 100 |
| ACIDE ASCORBIQUE | 0,1 à 10 |
| ADENOSINE | 0,01 à 1 |
| GUANINE | 0,01 à 1 |
| DEOXYRIBOSE | 0,01 à 1 |
| RIBOSE | 0,01 à 1 |
| CHOLINE CHLORIDE | 0 à 3 |
| MPC | 0 à 5000 |

Comme démontré ci-après, un tel milieu enrichi possède une capacité *in vitro* à stimuler la croissance des fibroblastes sur plusieurs jours. Par ailleurs, il permet une croissance des fibroblastes stimulée en présence de sérum. Il constitue donc un candidat particulièrement adapté dans le contexte d'une injection intra-articulaire, dans la mesure où une partie du liquide synovial est exfiltrée du plasma sanguin.

En outre et comme démontré dans la présente demande, l'incubation de fibroblastes dans ce milieu augmente la capacité de ces cellules à faire face au stress oxydatif, possédant ainsi des propriétés anti-radicalaires. Ainsi, *in vitro*, le milieu enrichi exerce un effet inhibiteur sur l'excès de production mitochondriale des espèces réactive de l'oxygène (ion superoxyde) par des fibroblastes exposés à un inhibiteur de la chaine respiratoire (antimycine A). La cinétique d'expression d'un témoin fluorescent d'oxydation (DCFDA) est également significativement réduite pour des fibroblastes humains pré-incubés dans le milieu de croissance et soumis à un choc oxydatif chimique (AAPH), par rapport aux fibroblastes témoins pré-incubés en milieu DMEM standard. Les propriétés antiradicalaires du milieu de croissance des fibroblastes lui confèrent ainsi également un rôle de protection de l'acide hyaluronique vis à vis de la dégradation radicalaire dans l'articulation, susceptible d'augmenter la rémanence de ce composé *in situ* et de nature à prolonger l'efficacité thérapeutique de la viscosupplémentation.

Ainsi, le « milieu de croissance des fibroblastes » selon l'invention qui peut être appelé «milieu naturel complet de survie et de croissance des fibroblastes », doit présenter les caractéristiques suivantes :
a/ de composition déterminée, traçable, ne comportant que des facteurs de croissance cellulaire naturellement présents dans l'organisme ou neutres vis à vis de celui-ci (acides aminés, peptides, vitamines, oligoéléments, sels minéraux, sucres simples et complexes, acides nucléiques), à l'exclusion de toute substance d'origine non naturelle, de composition non définie ou encore médicamenteuse ;
b/ qui permet d'une part, à lui seul, la survie des fibroblastes en culture ;
c/ et d'autre part stimule leur croissance et leur métabolisme (et donc leurs productions cellulaires).

Une composition selon l'invention peut en outre contenir d'autres ingrédients ou excipients, couramment employés dans cette application, notamment des dérivés ou des fractions purifiées de l'AH. Toutefois, selon un mode de réalisation particulier, la composition injectable ne comprend que les deux composants décrits ci-dessus : agent de viscosupplémentation, avantageusement de l'acide hyaluronique éventuellement combiné à un ou plusieurs autres polysaccharides d'origine naturelle d'une part, et milieu de culture de fibroblastes d'autre part.

Comme déjà dit, cette composition a pour vocation d'être injectée au niveau intra-articulaire, dans la cavité articulaire d'un sujet.

Au sens de la présente invention, le terme « sujet » désigne un mammifère, préférentiellement un humain, mais peut également désigner un animal soumis à un traitement vétérinaire, notamment les animaux de compagnie ou de loisir (par exemple chiens, chats ou chevaux).

A priori, toutes les articulations peuvent être traitées à l'aide d'une composition selon l'invention. Le genou est une articulation préférentiellement visée chez le sujet humain. Chez le chien, une articulation couramment traitée est la hanche, alors que chez le cheval, il s'agit avantageusement du carpe, du boulet ou du jarret.

Selon un mode de réalisation avantageux, la composition, avantageusement aqueuse, se présente sous la forme d'un gel, en raison de l'application injectable faisant l'objet de l'invention. De manière remarquable, cette contrainte est tout à fait compatible avec les milieux de croissance des fibroblastes décrits ci-dessus, qui sont formulables sous forme de gel, grâce à l'incorporation d'acide hyaluronique, sans ajout d'excipients exogènes.

Encore plus avantageusement, la composition se présente sous la forme d'un hydrogel monophasique, à savoir un hydrogel sous forme d'une seule phase homogène. La viscosité de la composition obtenue peut être aisément ajustée, notamment en adaptant la composition et la quantité de l'acide hyaluronique pour obtenir des propriétés rhéologiques similaires à celles du liquide synovial.

Ainsi et à titre d'exemple, il a été montré qu'une composition selon l'invention présentant une osmolarité comprise entre 300 et 350 mOsm, un pH compris entre 7,4 et 7,6 et une concentration en acide hyaluronique de poids moléculaire 1,3 à 1,8 MDa comprise entre 10 et 25 mg/ml était tout à fait compatible avec l'application visée.

La composition injectable selon l'invention peut également faire l'objet d'un kit qui comprend en outre des seringues pouvant contenir ladite composition. Il s'agit par exemple de seringues unidoses de 2 à 20 ml. Dans un tel kit, les 2 constituants essentiels de la composition peuvent être présentés en tant que mélange dans une même seringue, ou dans 2 seringues distinctes pour un mélange extemporané.

Etant donnés le caractère injectable et la visée thérapeutique d'une telle composition, celle-ci est avantageusement soumise à stérilisation, stérilisation ayant lieu avantageusement à froid de manière à ne pas dénaturer les composants en présence. Cette étape peut par exemple être réalisée par filtration sur membrane à 0,22 µm pour le milieu de croissance des fibroblastes, et par stérilisation séparée selon un processus connu de l'homme de l'art pour l'acide hyaluronique.

Une autre alternative consiste à proposer la composition injectable sous forme d'une poudre (AH et milieu de croissance des fibroblastes), en flacons de verre, de polypropylène, de polyéthylène ou tout autre matériau pouvant résister à une stérilisation par radiation ionisante ou par flash point thermique. Dans ce mode réalisation, l'hydrogel monophasique est reconstitué par addition d'eau stérile dans le flacon, à l'aide d'une seringue (stérile), avant l'injection du produit dans l'articulation. Dans ce cas, le produit doit être reconstitué dans les 2 à 72 heures avant l'injection.

Etant donné leur mode d'action complémentaire, les constituants de la composition selon l'invention peuvent être mélangés et/ou administrés de manière simultanée, séparée ou étalée dans le temps.

Une application directe de la composition selon l'invention est le traitement des dégénérescences articulaires, en particulier de l'arthrose.

Une composition selon l'invention est donc destinée à être utilisée en tant que dispositif médical et/ou médicament.

L'invention va maintenant être illustrée de manière non limitative par les exemples suivants, à l'appui des figures annexées.

### Légendes des figures

La figure 1 illustre la croissance comparée de fibroblastes humains en culture dans un milieu de croissance des fibroblastes selon l'invention et le milieu standard DMEM (sigma), sans facteur de croissance.
La figure 2 représente les phénomènes d'oxydation mesurés dans une culture de fibroblastes humains exposés à un stress oxydatif après incubation dans différents milieux.

### Exemples de réalisation

### 1/ Utilisation d'un milieu de croissance des fibroblastes dans une composition injectable :

### a) Composition du milieu :

| DENOMINATION INTERNATIONALE NOMENCLATURE COSMETIQUE INGREDIENT (INCI) | *CONCENTRATION FINALE* Solution 1 X (en mg/l) |
|---|---|
| EAU | q.s.p. 1 litre |
| CHLORURE DE SODIUM | 5000 à 8000 |
| L-GLUTAMINE ou L-ALANYL-GLUTAMINE | 100 à 3000 |
| SODIUM BICARBONATE | 0 à 2000 |
| D-GLUCOSE | 2000 à 5000 |
| L-ARGININE HCL | 300 à 500 |
| SODIUM ACETATE | 200 à 450 |
| DISODIUM PHOSPHATE Na₂HPO4 | 100 à 1500 |
| L-LEUCINE | 50 à 200 |
| L-SERINE | 50 à 200 |
| CHLORURE DE MAGNESIUM MgCl₂, 6H₂O | 50 à 200 |
| CHLORURE DE POTASSIUM | 50 à 200 |
| L-VALINE | 20 à 150 |
| SODIUM PYRUVATE | 10 à 75 |
| L-LYSINE HCL | 10 à 75 |
| L-HISTIDINE HCL,H₂O | 10 à 75 |
| L-CYSTEINE HCL H₂O | 10 à 75 |
| ADENINE (HCl) | 5 à 50 |
| L-THREONINE | 5 à 50 |
| CHLORURE DE CALCIUM CaCl₂, 2H₂O | 0 à 22,5 |
| MYO-INOSITOL | 5 à 50 |
| ACIDE L-GLUTAMIQUE | 15 à 75 |
| L-ASPARAGINE H₂O | 15 à 75 |
| L-METHIONINE | 10 à 50 |
| L-TYROSINE 2Na₂ 2H₂O | 10 à 50 |
| L-PHENYLALANINE | 2 à 20 |
| L-TRYPTOPHANE | 2 à 20 |
| L-ALANINE | 5 à 30 |
| GLYCINE | 5 à 30 |
| L-ISOLEUCINE | 5 à 30 |
| ACIDE L-ASPARTIQUE | 10 à 50 |
| SODIUM SULFATE | 1 à 10 |
| SULFATE FERREUX FeSO₄, 7H₂O | 1 à 10 |
| ACIDE FOLIQUE | 1 à 5 |
| THYMIDINE | 0,1 à 3 |
| CYANOCOBALAMINE | 0,1 à 3 |
| D-CALCIUM PANTOTHENATE | 1 à 5 |
| THIAMINE HCL | 1 à 5 |
| ACIDE THIOCTIQUE | 0,1 à 1 |
| ZINC SULFATE ZnSO₄,7H₂O | 0,05 à 0,5 |
| SODIUM SILICATE NA₂SIO₃,4H₂O | 0,05 à 0,5 |
| PYRIDOXINE HCL | 0,5 à 3 |
| NIACINAMIDE (NICOTINAMIDE) | 0,5 à 3 |
| RIBOFLAVINE | 0,05 à 0,5 |
| d-BIOTIN | 0,01 à 0,05 |
| SULFATE DE CUIVRE CuSO₄,5H₂O | 0 à 0,005 |
| AMMONIUM MOLYBDATE (NH4)₆MO7O₂₄,4H₂O | 0 à 0,005 |
| AMMONIUM VANADATE NH₄VO₃ | 0 à 0,001 |
| CHLORURE DE MANGANESE MnCl₂, 4H₂O | 0 à 0,0001 |
| HYALURONATE DE SODIUM | 100 à 1000 |
| L-PROLINE | 10 à 100 |
| HYDROXYPROLINE | 10 à 100 |
| ACIDE ASCORBIQUE | 0,1 à 10 |
| ADENOSINE | 0,01 à 1 |
| GUANINE | 0,01 à 1 |
| DEOXYRIBOSE | 0,01 à 1 |
| RIBOSE | 0,01 à 1 |
| CHOLINE CHLORIDE | 0 à 3 |
| MPC | 0 à 5000 |

### b) Culture de fibroblastes humains

### Protocole

- Fibroblastes humains ensemencés à faible densité en plaques de 96 puits sous milieu de culture standard DMEM, supplémenté en facteur de croissance cellulaire SVF (Sérum de Veau Foetal) ;
- Après 24h, culture dans le milieu selon l'invention pur ou dans le milieu standard DMEM sans facteur de croissance ;
- Absence de renouvellement des milieux au cours de l'expérience ;
- Densité des cellules vivantes évaluée à T0 puis après 2, 4, 7 et 9 jours, par méthode colorimétrique (réactif WST-1).

### Résultats

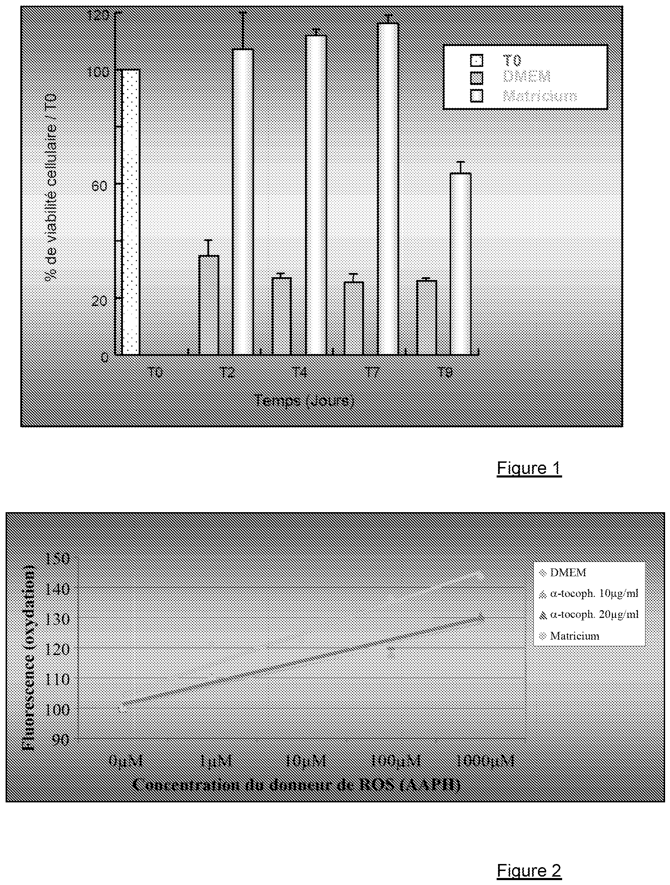

- Le milieu de culture selon l'invention permet de soutenir, seul, la croissance des fibroblastes sur une durée de 9 jours. On observe à partir du 7ème jour un ralentissement de la croissance cellulaire s'expliquant par le non renouvellement du milieu (Fig. 1).
- En milieu DMEM sans SVF, on observe une diminution de la viabilité cellulaire au bout de 2 jours et l'absence de croissance cellulaire tout au long de l'étude (Fig. 1).

En conclusion, il ressort que le milieu de croissance des fibroblastes mis en oeuvre selon l'invention permet la survie et stimule la croissance de fibroblastes humains normaux en l'absence de facteurs de croissance exogènes.

### c) Augmentation de la résistance des fibroblastes au stress oxydatif

### Protocole

• Fibroblastes humains normaux en phase de croissance ensemencés dans des plaques de 96 puits, en milieu DMEM complémenté (10% sérum de veau foetal).
• 48h après, placement en milieu DMEM (contrôle négatif), dans le milieu selon l'invention, ou α-tocophérol (contrôle anti-oxydant positif) pendant 2h.
• Rinçage et incubation 25 min avec un donneur massif de ROS (choc oxydatif par l'AAPH) et un révélateur (DCFDA) qui devient fluorescent lorsqu'il est oxydé.
• Vitesse d'apparition de la fluorescence (proportionnelle à la production de ROS cellulaire) mesurée toutes les 3 minutes.

### Résultats

• Inhibition significative de la fluorescence (oxydation) pour les cellules pré-incubées dans le milieu selon l'invention (Matricium) par rapport au témoin négatif DMEM (Fig. 2). Le milieu selon l'invention augmente la capacité des cellules à faire face au choc oxydatif.
• Effet inhibiteur indirect du stress oxydatif (amélioration de l'homéostasie redox des cellules) comparable en intensité à celui d'un anti-oxydant à effet classique direct (α-tocophérol capteur des radicaux libres).

En conclusion, il ressort que ce milieu exerce un effet inhibiteur sur l'excès de production mitochondriale des espèces réactive de l'oxygène (ion superoxyde) par des fibroblastes exposés à un inhibiteur de la chaine respiratoire (antimycine A). La cinétique d'expression d'un témoin fluorescent d'oxydation (DCFDA) est également significativement réduite pour des fibroblastes humains pré-incubés dans le milieu de croissance et soumis à un choc oxydatif chimique (AAPH), par rapport aux fibroblastes témoins pré-incubés en milieu DMEM standard.

### 2/Préparation d'un gel injectable pour injection intra-articulaire :

- Milieu de croissance des fibroblastes.
- HA ajouté à une concentration comprise entre 1 et 100 mg/ml et préférentiellement à une concentration comprise entre 10 et 25 mg/ml.
- Formulation d'un gel : l'acide hyaluronique (AH) est mis en solution dans le milieu de culture des fibroblastes. La concentration en AH détermine la viscosité de la préparation finale. A titre d'exemple, l'AH utilisé est du hyaluronate de sodium dont le poids moléculaire est compris entre 1,3 et 1,8 MDa. Le gel injectable selon l'invention ne comporte aucun additif, l'ensemble des composants de la formule jouant à la fois le rôle d'excipients et d'ingrédients actifs.
- Stérilisation : par filtration sur membrane à 0,22 µm pour le milieu de croissance des fibroblastes, et par stérilisation séparée selon un processus connu de l'homme de l'art pour l'HA. Une autre alternative consiste à proposer la composition injectable sous forme d'une poudre (AH et milieu de croissance des fibroblastes), en flacons résistants à une stérilisation par radiation ionisante ou par flash point thermique. L'hydrogel monophasique est reconstitué par addition d'eau stérile dans le flacon, avant injection du produit dans l'articulation.
- Protocole d'injection : selon l'articulation à traiter et la sévérité de l'arthrose, il est préconisé de réaliser une injection par semaine pendant 3 à 5 semaines. La durée d'action est fonction de l'importance des lésions articulaires et de l'âge du sujet.

## Revendications

1. Composition injectable par voie intra-articulaire comprenant :
- au moins un agent de viscosupplémentation choisi dans le groupe : acide hyaluronique, chondroïtine sulfate, kératane, kératane sulfate, héparine, cellulose et ses dérivés, chitosane, xanthanes, galactomannane, alginates et leurs sels respectifs ; et
- un milieu de croissance des fibroblastes exempt de tout facteur de croissance cellulaire et exempt de tout extrait biologique d'origine animale ou cellulaire, non tracé et/ou de composition indéterminée, constitué de :
Chlorure de sodium à une concentration finale comprise entre 5000 et 8000 mg/l ;
L-glutamine ou L-alanyl-glutamine à une concentration finale comprise entre 100 et 3000 mg/l ;
Sodium bicarbonate à une concentration finale comprise entre 0 et 2000 mg/l ;
D-glucose à une concentration finale comprise entre 2000 et 5000 mg/l ;
L-arginine HCl à une concentration finale comprise entre 300 et 500 mg/l ;
Sodium acétate à une concentration finale comprise entre 200 et 450 mg/l ;
Disodium phosphate (Na₂HPO4) à une concentration finale comprise entre 100 et 1500 mg/l ;
L-leucine à une concentration finale comprise entre 50 et 200 mg/l ;
L-sérine à une concentration finale comprise entre 50 et 200 mg/l ;
Chlorure de magnésium (MgCl₂, 6H₂O) à une concentration finale comprise entre 50 et 200 mg/l ;
Chlorure de potassium à une concentration finale comprise entre 50 et 200 mg/l ;
L-valine à une concentration finale comprise entre 20 et 150 mg/l ;
Sodium pyruvate à une concentration finale comprise entre 10 et 75 mg/l ;
L-lysine HCl à une concentration finale comprise entre 10 et 75 mg/l ;
L-histidine HCl,H₂O à une concentration finale comprise entre 10 et 75 mg/l ;
L-cystéine HCl H₂O à une concentration finale comprise entre 10 et 75 mg/l ;
Adénine (HCl) à une concentration finale comprise entre 5 et 50 mg/l ;
L-thréonine à une concentration finale comprise entre 5 et 50 mg/l ;
Chlorure de calcium (CaCl₂, 2H₂O) à une concentration finale comprise entre 0 et 22,5 mg/l ;
Myo- inositol à une concentration finale comprise entre 5 et 50 mg/l ;
Acide L-glutamique à une concentration finale comprise entre 15 et 75 mg/l ;
L-asparagine H₂O à une concentration finale comprise entre 15 et 75 mg/l ;
L-méthionine à une concentration finale comprise entre 10 et 50 mg/l ;
L-tyrosine Na₂ 2H₂O à une concentration finale comprise entre 10 et 50 mg/l ;
L-phénylalanine à une concentration finale comprise entre 2 et 20 mg/l ;
L-tryptophane à une concentration finale comprise entre 2 et 20 mg/l ;
L-alanine à une concentration finale comprise entre 5 et 30 mg/l ;
Glycine à une concentration finale comprise entre 5 et 30 mg/l ;
L-isoleucine à une concentration finale comprise entre 5 et 30 mg/l ;
Acide L-aspartique à une concentration finale comprise entre 10 et 50 mg/l ;
Sodium sulfate à une concentration finale comprise entre 1 et 10 mg/l ;
Sulfate ferreux (FeSO₄,7H₂O) à une concentration finale comprise entre 1 et 10 mg/l ;
Acide folique à une concentration finale comprise entre 1 et 5 mg/l ;
Thymidine à une concentration finale comprise entre 0,1 et 3 mg/l ;
Cyanocobalamine à une concentration finale comprise entre 0,1 et 3 mg/l ;
D-calcium pantothénate à une concentration finale comprise entre 1 et 5 mg/l ;
Thiamine HCL à une concentration finale comprise entre 1 et 5 mg/l ;
Acide thioctique à une concentration finale comprise entre 0,1 et 1 mg/l ;
Zinc sulfate (ZnSO₄,7H₂O) à une concentration finale comprise entre 0,05 et 0,5 mg/l ;
Sodium silicate (Na₂SIO₃,4H₂O) à une concentration finale comprise entre 0,05 et 0,5 mg/l ;
Pyridoxine HCL à une concentration finale comprise entre 0,5 et 3 mg/l ;
Niacinamide (nicotinamide) à une concentration finale comprise entre 0,5 et 3 mg/l ;
Riboflavine à une concentration finale comprise entre 0,05 et 0,5 mg/l ;
D-biotine à une concentration finale comprise entre 0,01 et 0,05 mg/l ;
Sulfate de cuivre (CuSO₄,5H₂O) à une concentration finale comprise entre 0 et 0,005 mg/l ;
Ammonium molybdate ((NH4)₆MO7O₂₄,4H₂O) à une concentration finale comprise entre 0 et 0,005 mg/l ;
Ammonium vanadate (NH₄VO₃) à une concentration finale comprise entre 0 et 0,001 mg/l ;
Chlorure de manganèse (MnCl₂, 4H₂O) à une concentration finale comprise entre 0 et 0,0001 mg/l ;
Hyaluronate de sodium à une concentration finale comprise entre 100 et 1000 mg/l ;
L-proline à une concentration finale comprise entre 10 et 100 mg/l ;
Hydroxyproline à une concentration finale comprise entre 10 et 100 mg/l ;
Acide ascorbique à une concentration finale comprise entre 0,1 et 10 mg/l ;
Adénosine à une concentration finale comprise entre 0,01 et 1 mg/l ;
Guanine à une concentration finale comprise entre 0,01 et 1 mg/l ;
Déoxyribose à une concentration finale comprise entre 0,01 et 1 mg/l ;
Ribose à une concentration finale comprise entre 0,01 et 1 mg/l ;
Choline chloride à une concentration finale comprise entre 0 et 3 mg/l ;
Mélange de peptides de lait (MPC) à une concentration finale comprise entre 0 et 5000 mg/l ;
Eau qsp 1 litre.

2. Composition selon la revendication 1, ***caractérisée* en ce qu'**elle est constituée de :
- l'acide hyaluronique ou l'un de ses sels en tant qu'agent de viscosupplémentation ; et
- le milieu de croissance des fibroblastes.

3. Composition selon la revendication 1, ***caractérisée* en ce qu'**elle comprend :
- l'acide hyaluronique ou l'un de ses sels en tant qu'agent de viscosupplémentation ;
- au moins un autre polysaccharide, avantageusement d'origine naturelle, et encore plus avantageusement choisi dans le groupe de : la chondroïtine sulfate, le kératane, le kératane sulfate, l'héparine, la cellulose et ses dérivés, le chitosane, les xanthanes, le galactomannane, les alginates et leurs sels respectifs ; et
- le milieu de croissance des fibroblastes.

4. Composition selon la revendication 3, ***caractérisée* en ce qu'**elle est constituée de :
- l'acide hyaluronique ou l'un de ses sels en tant qu'agent de viscosupplémentation ;
- au moins un autre polysaccharide, avantageusement d'origine naturelle, et encore plus avantageusement choisi dans le groupe : la chondroïtine sulfate, le kératane, le kératane sulfate, l'héparine, la cellulose et ses dérivés, le chitosane, les xanthanes, le galactomannane, les alginates et leurs sels respectifs ; et
- le milieu de croissance des fibroblastes.

5. Composition selon l'une des revendications précédentes, ***caractérisée* en ce qu'**elle se présente sous la forme d'un gel, avantageusement un gel aqueux stérile.

6. Composition selon l'une des revendications précédentes, ***caractérisée* en ce que** l'agent de viscosupplémentation, avantageusement l'acide hyaluronique, est présent dans la composition à une concentration comprise entre 1 et 100 mg/ml, avantageusement comprise entre 10 et 25 mg/ml.

7. Kit se présentant sous la forme de seringues et contenant une composition selon l'une des revendications 1 à 6.

8. Dispositif médical comprenant une composition selon l'une des revendications 1 à 6.

9. Composition selon l'une des revendications 1 à 6 ou kit selon la revendication 7 ou dispositif médical selon la revendication 8 pour utilisation dans le traitement des dégénérescences articulaires, en particulier de l'arthrose.

10. Produits contenant un agent de viscosupplémentation et un milieu de croissance des fibroblastes tels que définis dans l'une des revendications 1 à 6 comme produit de combinaison pour une utilisation simultanée, séparée ou étalée dans le temps, pour le traitement des dégénérescences articulaires, en particulier de l'arthrose.

## Patentansprüche

1. Injizierbare Zusammensetzung zur intraartikulären Verwendung, umfassend:
- eine Viskosupplementation, die aus der Gruppe von Hyaluronsäure, Chondroitinsulfat, Keratan, Keratansulfat, Heparin, Zellulose und ihren Derivate, Chitosan, Xanthane, Galactomannane, Alginate und ihren jeweiligen Salzen ausgewählt wird; und
- ein Wachstumsmedium für Fibroblasten, das frei von jedem Zellwachstumsfaktor und frei von jedem biologischen Extrakt tierischen oder Zellursprungs ist, das nicht gekennzeichnet ist und/oder mit unbestimmter Zusammensetzung, bestehend aus:
Natriumchlorid, mit einer Endkonzentration zwischen 5000 und 8000 mg/l;
L-Glutamin oder L-Alanyl-Glutamin mit einer Endkonzentration zwischen 100 und 3000 mg/l;
Natriumbicarbonat mit einer Endkonzentration zwischen 0 und 2000 mg/l;
D- Glucose mit einer Endkonzentration zwischen 2000 und 5000 mg/l;
L- Arginin HCl mit einer Endkonzentration zwischen 300 und 500 mg/l;
Natriumacetat mit einer Endkonzentration zwischen 200 und 450 mg/l;
Di-Natriumphosphat (Na₂HPO₄), mit einer Endkonzentration zwischen 100 und 1500 mg/l;
L-Leucin mit einer Endkonzentration zwischen 50 und 200 mg/l;
L-Serin mit einer Endkonzentration zwischen 50 und 200 mg/l;
Magnesiumchlorid (MgCl₂, 6H₂O), mit einer Endkonzentration zwischen 50 und 200 mg/l;
Kaliumchlorid mit einer Endkonzentration zwischen 50 und 200 mg/l;
L-Valin mit einer Endkonzentration zwischen 20 und 150 mg/l;
Natriumpyruvat mit einer Endkonzentration zwischen 10 und 75 mg/l;
L-Lysin HCl mit einer Endkonzentration zwischen 10 und 75 mg/l;
L-Histidin HCl H₂O mit einer Endkonzentration zwischen 10 und 75 mg/l;
L-Cystein HCl H₂O mit einer Endkonzentration zwischen 10 und 75 mg/l;
Adenin (HCl) mit einer Endkonzentration zwischen 5 und 50 mg/l;
L-Threonin mit einer Endkonzentration zwischen 5 und 50 mg/l;
Kalziumchlorid (CaCl₂, H₂O) mit einer Endkonzentration zwischen 0 und 22,5 mg/l;
Myo-inositol mit einer Endkonzentration zwischen 5 und 50 mg/l;
L-Glutaminsäure mit einer Endkonzentration zwischen 15 und 75 mg/l;
L-Asparagin H₂O mit einer Endkonzentration zwischen 15 und 75 mg/l;
L-Methionin, mit einer Endkonzentration zwischen 10 und 50 mg/l;
L-Tyrosin Na₂ 2H₂O, mit einer Endkonzentration zwischen 10 und 50 mg/l;
L-Phenylalanin mit einer Endkonzentration zwischen 2 und 20 mg/l;
L-Tryptophan mit einer Endkonzentration zwischen 2 und 20 mg/l;
L-Alanin mit einer Endkonzentration zwischen 5 und 30 mg/l;
Glycin, mit einer Endkonzentration zwischen 5 und 30 mg/l;
L-Isoleucin mit einer Endkonzentration zwischen 5 und 30 mg/l;
L-Aspartinsäure mit einer Endkonzentration zwischen 10 und 50 mg/l;
Natriumsulfat, mit einer Endkonzentration zwischen 1 und 10 mg/l;
Eisensulfat (FeSO₄,7H₂O), mit einer Endkonzentration zwischen 1 und 10 mg/l;
Folsäure mit einer Endkonzentration zwischen 1 und 5 mg/l;
Thymidin mit einer Endkonzentration zwischen 0,1 und 3 mg/l;
Cyanocobalamin mit einer Endkonzentration zwischen 0,1 und 3 mg/l;
D-Kalzium-Pantothenat mit einer Endkonzentration zwischen 1 und 5 mg/l;
Thiamin HCl mit einer Endkonzentration zwischen 1 und 5 mg/l;
Thioctsäure mit einer Endkonzentration zwischen 0,1 und 1 mg/l;
Zinksulfat (ZnSO₄, 7H₂O), mit einer Endkonzentration zwischen 0,05 und 0,5 mg/l;
Natriumsilicat (Na₂SIO₃, 4H₂O) mit einer Endkonzentration zwischen 0,05 und 0,5 mg/l;
Pyridoxin HCl mit einer Endkonzentration zwischen 0,5 und 3 mg/l;
Niacinamid (Nicotinamid) mit einer Endkonzentration zwischen 0,5 und 3 mg/l;
Riboflavin mit einer Endkonzentration zwischen 0,05 und 0,5 mg/l;
D-Biotin mit einer Endkonzentration zwischen 0,01 und 0,05 mg/l;
Kupfersulfat (CuSO₄, 5H₂O) mit einer Endkonzentration zwischen 0 und 0,005 mg/l ;
Ammoniummolybdat (NH4)₆MO₇O₂₄, 4H₂O) mit einer Endkonzentration zwischen 0 und 0,005 mg/l ;
Ammoniumvanadat (NH₄VO₃) mit einer Endkonzentration zwischen 0 und 0,001 mg/l;
Manganchlorid (MnCl₂, 4H₂O) mit einer Endkonzentration zwischen 0 und 0,0001 mg/l;
Natriumhyaluronat mit einer Endkonzentration zwischen 100 und 1000 mg/l;
L-Prolin mit einer Endkonzentration zwischen 10 und 100 mg/l;
Hydroxyprolin mit einer Endkonzentration zwischen 10 und 100 mg/l;
Ascorbinsäure mit einer Endkonzentration zwischen 0,1 und 10 mg/l;
Adenosin mit einer Endkonzentration zwischen 0,01 und 1 mg/l;
Guanin mit einer Endkonzentration zwischen 0,01 und 1 mg/l;
Desoxyribose mit einer Endkonzentration zwischen 0,01 und 1 mg/l;
Ribose mit einer Endkonzentration zwischen 0,01 und 1 mg/l;
Cholinchlorid mit einer Endkonzentration zwischen 0 und 3 mg/l;
Mischung aus Milchpeptiden (MPCI), mit einer Endkonzentration zwischen 0 und 5000 mg/l;
Wasser q.s.p. 1 Liter.

2. Zusammensetzung gemäß Anspruch 1, **dadurch gekennzeichnet, dass** sie besteht aus:
- Hyaluronsäure oder einem ihrer Salze als Viskosupplementation; sowie
- dem Wachstumsmedium für Fibroblasten.

3. Zusammensetzung gemäß Anspruch 1, **dadurch gekennzeichnet, dass** sie umfasst:
- Hyaluronsäure oder einem ihrer Salze als Viskosupplementation;
- mindestens einem weiteren Polysaccharid, vorteilhafterweise natürlicher Herkunft und noch besser, das aus der Gruppe von Chondroitinsulfat, Keratan, Keratansulfat, Heparin, Zellulose und ihren Derivaten, Chitosan, Xanthane, Galactomannane, Alginate und ihren jeweiligen Salzen ausgewählt wird; und
- das Wachstumsmedium für Fibroblasten.

4. Zusammensetzung gemäß Anspruch 1, **dadurch gekennzeichnet, dass** sie besteht aus:
- Hyaluronsäure oder einem ihrer Salze als Viskosupplementation;
- mindestens einem weiteren Polysaccharid, vorteilhafterweise natürlicher Herkunft und noch besser, das aus der Gruppe von Chondroitinsulfat, Keratan, Keratansulfat, Heparin, Zellulose und ihren Derivaten, Chitosan, Xanthane, Galactomannane, Alginate und ihren jeweiligen Salzen ausgewählt wird; und
- das Wachstumsmedium für Fibroblasten.

5. Zusammensetzung gemäß Anspruch 1, **dadurch gekennzeichnet, dass** sie die Form eines Gels, am besten eines sterilen, wässrigen Gels, hat.

6. Zusammensetzung gemäß einem der vorhergehenden Ansprüche, **dadurch gekennzeichnet, dass** die Viskosupplementation, am besten Hyaluronsäure, in der Zusammensetzung in einer Konzentration zwischen 1 und 100 mg/ml, am besten zwischen 10 und 25 mg/ml, vorhanden ist.

7. Set in Form von Spritzen, das eine Zusammensetzung gemäß einem der Ansprüche 1 bis 6 enthält.

8. Medizinprodukt mit einer Zusammensetzung gemäß einem der Ansprüche 1 bis 6.

9. Zusammensetzung nach einem der Ansprüche 1 bis 6, oder Set nach Anspruch 7 oder Medizinprodukt gemäß Anspruch 8, zum Einsatz in der Behandlung von Gelenkdegenerationen, insbesondere Arthrose.

10. Produkte mit einer Viskosupplementation und einem Wachstumsmedium für Fibroblasten wie definiert in einem der Ansprüche 1 bis 6, als Kombinationsprodukt zur gleichzeitigen, getrennten oder zeitlich versetzten Verwendung, zur Behandlung von Gelenkdegenerationen, insbesondere Arthrose.

## Claims

1. A composition for intra-articular injection, comprising:
- at least one viscosupplementation agent selected from the group consisting of hyaluronic acid, chondroitin sulphate, keratan, keratan sulphate, heparin, cellulose and its derivatives, chitosan, xanthans, galactomannan, alginates and their salts; and
- a fibroblast growth medium free of any cell growth factor and free of any biological extract of animal or cellular origin which has not been traced and/or is of undefined composition, composed of:
Sodium chloride at a final concentration between 5000 and 8000 mg/l ;
L-glutamine or L-alanyl-glutamine at a final concentration between 100 and 3000 mg/l;
Sodium bicarbonate at a final concentration between 0 and 2000 mg/l ;
D-glucose at a final concentration between 2000 and 5000 mg/l;
L-arginine HCl at a final concentration between 300 and 500 mg/l;
Sodium acetate at a final concentration between 200 and 450 mg/l;
Disodium phosphate (Na₂HPO₄) at a final concentration between 100 and 1500 mg/l;
L-leucine at a final concentration between 50 and 200 mg/l ;
L-serine at a final concentration between 50 and 200 mg/l;
Magnesium chloride (MgCl₂, 6H₂O) at a final concentration between 50 and 200 mg/l ;
Potassium chloride at a final concentration between 50 and 200 mg/l ;
L-valine at a final concentration between 20 and 150 mg/l ;
Sodium pyruvate at a final concentration between 10 and 75 mg/l ;
L-lysine HCl at a final concentration between 10 and 75 mg/l ;
L-histidine HCl, H₂O at a final concentration between 10 and 75 mg/l ;
L-cysteine HCl, H₂O at a final concentration between 10 and 75 mg/l ;
Adenine (HCl) at a final concentration between 5 and 50 mg/l ;
L-threonine at a final concentration between 5 and 50 mg/l ;
Calcium chloride (CaCl₂, 2H₂O) at a final concentration between 0 and 22.5 mg/l ;
Myo-inositol at a final concentration between 5 and 50 mg/l ;
L-glutamic acid at a final concentration between 15 and 75 mg/l ;
L-asparagine H₂O at a final concentration between 15 and 75 mg/l ;
L-methionine at a final concentration between 10 and 50 mg/l ;
L-tyrosine Na₂, 2H₂O at a final concentration between 10 and 50 mg/l ;
L-phenylalanine at a final concentration between 2 and 20 mg/l ;
L-tryptophan at a final concentration between 2 and 20 mg/l ;
L-alanine at a final concentration between 5 and 30 mg/l ;
Glycine at a final concentration between 5 and 30 mg/l ;
L-isoleucine at a final concentration between 5 and 30 mg/l ;
L-aspartic acid at a final concentration between 10 and 50 mg/l ;
Sodium sulphate at a final concentration between 1 and 10 mg/l ;
Ferrous sulphate (FeSO₄, 7H₂O) at a final concentration between 1 and 10 mg/l ;
Folic acid at a final concentration between 1 and 5 mg/l ;
Thymidine at a final concentration between 0.1 and 3 mg/l ;
Cyanocolalamin at a final concentration between 0.1 and 3 mg/l ;
D-calcium pantothenate at a final concentration between 1 and 5 mg/l ;
Thiamine HCl at a final concentration between 1 and 5 mg/l ;
Thioctic acid at a final concentration between 0.1 and 1 mg/l ;
Zinc sulphate (ZnSO₄, 7H₂O) at a final concentration between 0.05 and 0.5 mg/l ;
Sodium silicate (Na₂SIO₃, 4H₂O) at a final concentration between 0.05 and 0.5 mg/l ;
Pyridoxine HCl at a final concentration between 0.5 and 3 mg/l ;
Niacinamide (nicotinamide) at a final concentration between 0.5 and 3 mg/l ;
Riboflavin at a final concentration between 0.05 and 0.5 mg/l ;
D-biotin at a final concentration between 0.01 and 0.05 mg/l ;
Copper sulphate (CuSO₄, 5H₂O) at a final concentration between 0 and 0.005 mg/l ;
Ammonium molybdate ((NH₄)₆MO₇O₂₄, 4H₂O) at a final concentration between 0 and 0.005 mg/l ;
Ammonium vanadate (NH₄VO₃) at a final concentration between 0 and 0.001 mg/l ;
Manganese chloride (MnCl₂, 4H₂O) at a final concentration between 0 and 0.0001 mg/l ;
Sodium hyaluronate at a final concentration between 100 and 1000 mg/l ;
L-proline at a final concentration between 10 and 100 mg/l ;
Hydroxyproline at a final concentration between 10 and 100 mg/l ;
Ascorbic acid at a final concentration between 0.1 and 10 mg/l ;
Adenosine at a final concentration between 0.01 and 1 mg/l ;
Guanine at a final concentration between 0.01 and 1 mg/l ;
Desoxyribose at a final concentration between 0.01 and 1 mg/l ;
Ribose at a final concentration between 0.01 and 1 mg/l ;
Choline chloride at a final concentration between 0 and 3 mg/l ;
Milk peptide complex (MPC) at a final concentration between 0 and 5000 mg/l ;
Water qsp 1 litre.

2. A composition according to claim 1, **characterized in that** it is composed of:
- hyaluronic acid or a salt thereof as viscosupplementation agent; and
- the fibroblast growth medium.

3. A composition according to claim 1, **characterized in that** it comprises:
- hyaluronic acid or a salt thereof as viscosupplementation agent;
- at least one other polysaccharide, preferably of natural origin, more preferably selected from the group consisting of chondroitin sulphate, keratan, keratan sulphate, heparin, cellulose and its derivatives, chitosan, xanthans, galactomannan, alginates and their salts ; and
- the fibroblast growth medium.

4. A composition according to claim 3, **characterized in that** it is composed of:
- hyaluronic acid or a salt thereof as viscosupplementation agent;
- at least one other polysaccharide, preferably of natural origin, more preferably selected from the group consisting of chondroitin sulphate, keratan, keratan sulphate, heparin, cellulose and its derivatives, chitosan, xanthans, galactomannan, alginates and their salts ; and
- the fibroblast growth medium.

5. A composition according to one of the preceding claims, **characterized in that** it is in the form of a gel, preferably a sterile aqueous gel.

6. A composition according to one of the preceding claims, **characterized in that** the viscosupplementation agent, preferably hyaluronic acid, is present in the composition at a concentration of between 1 and 100 mg/ml, preferably between 10 and 25 mg/ml.

7. A kit in the form of syringes and containing a composition according to one of claims 1 to 6.

8. A medical device comprising the composition according to one of claims 1 to 6.

9. A composition according to one of claims 1 to 6 or a kit according to claim 7 or a medical device according to claim 8 for use to treat articular degeneration, preferably osteoarthritis.

10. Products containing a viscosupplementation agent and a fibroblast growth medium as defined in one of claims 1 to 6 as a combination product for simultaneous, separate or sequential use to treat articular degeneration; preferably osteoarthritis.
